# EUROPEAN PATENT APPLICATION

(11) **EP 1 559 705 A1**
(43) Date of publication of application: **03.08.2005**
(21) Application number: 03799169.2
(22) Date of filing: 30.09.2003
(51) Int. Cl.: C07C 51/15, C07C 65/03

(54) **PROCESS FOR PRODUCTION OF HYDROXYBENZOIC ACIDS**

(30) Priority: 01.10.2002 JP 2002288732
(71) Applicant: KABUSHIKI KAISHA UENO SEIYAKU OYO KENKYUJO, Osaka-shi, Osaka 541-0043 (JP)
(72) Inventor: UENO, Ryuzo, Nishinomiya-shi, Hyogo 662-0038 (JP); KITAYAMA, Masaya, Takarazuka-shi, Hyogo 665-0881 (JP); WAKAMORI, Hiroyuki, Hikami-gun, Hyogo 669-3105 (JP); YONETANI, Nobuhiro, Nishinomiya-shi, Hyogo 663-8022 (JP); HISANO, Takaya, 11, Ueno Seiyaku Dainishikoryo, Nishinomiya-shi, Hyogo 662-0066 (JP)
(74) Representative: Albrecht, Thomas, Dr.
(86) International application number: PCT/JP2003/012458
(87) International publication number: WO 2004/031113

(57) **Abstract**

The present invention provides a method for producing a hydroxybenzoic acid compound comprising, dehydrating a phenol compound and an alkaline metal compound to form the alkaline metal salt of the phenol compound and reacting the alkaline metal salt and carbon dioxide, wherein the dehydrating step is conducted by reacting the alkaline metal compound with an excess amount of the phenol compound at a temperature of 160°C or above. According to the method of the present invention, the hydroxybenzoic acid compound can be obtained by simple steps with low cost without using an expensive aprotic polar organic solvent. The provided method can produce highly pure hydroxybenzoic acid compound comprising little by-products in high yield.

## Description

### FIELD OF THE INVENTION

The present invention relates to a method for preparing a hydroxybenzoic acid compound.

### BACKGROUND OF THE INVENTION

As a method for preparing a hydroxybenzoic acid compound, a solid-gas phase reaction to react phenol potassium with carbon dioxide, which is known as Kolbe-Schmitt reaction, has conventionally been used. The Kolbe-Schmitt reaction, however, has some problems such as the long reaction time which is required due to the solid-gas phase reaction, the great loss of the reaction material because of side reaction due to the thermal non-uniformity of the reaction and the fluctuate of the yield because of the difficulty in controlling the reaction. In recent years, in order to overcome those problems of the solid-gas phase Kolbe-Schmitt reaction, liquid-phase methods wherein the reaction is conducted in a solvent or as slurry are proposed from the industrial viewpoints.

For example, Japanese Patent Application Laid Open No. 3-90047 discloses a method for preparing 3,5-dialkyl salicylic acid comprising heating 2,4-dialkylphenol and alkaline metal hydroxide in a mixed solvent of hydrocarbon and 1,3-dimethyl-2-imidazolidinone to give anhydrous 2,4-dialkylphenol alkaline metal salt through azeotropic dehydration and reacting said metal salt with carbon dioxide in said mixed solvent to give 3,5-dialkyl salicylic acid.

Though the use of an aprotic polar organic solvent such as 1,3-dimethyl-2-imidazolidinone in the reaction makes it possible to attain a high reaction yield, said method has some problems in collecting the product from the reaction mixture and recovering the solvent. That is, despite of the high reaction yield, 3,5-dialkyl salicylic acid alkaline metal salt can not be sufficiently collected by means of crystallization because of the high solubility of the alkaline metal salt to the aprotic polar organic solvent. In addition, though the aqueous solution of 3,5-dialkyl salicylic acid alkaline metal salt obtained by the reaction contains a large amount of aprotic polar organic solvent, the expensive aprotic polar organic solvent is difficult to collect because it transfers into the filtrate after the acid precipitation method.

In order to solve the above problems, Japanese Patent Application Laid Open No. 10-231271 proposes a method for preparing a hydroxybenzoic acid compound wherein an aprotic polar organic solvent is used as reaction solvent for the reaction between a phenol compound and an alkaline metal compound, characterized in that the molar ratio of the phenol compound to the total amount of the alkaline metal compound and the aprotic polar organic solvent is larger than 1.

However, the yield of hydroxybenzoic acid obtained by said method, which uses an excess amount of the phenol compound over the total amount of the alkaline metal compound and the aprotic polar solvent, is not sufficient. There is also a problem of side product such as a dimer of the phenol compound produced during the Kolbe-Schmitt reaction in the presence of an aprotic polar organic solvent and therefore, it is difficult to obtain high-purity hydroxybenzoic acids.

Furthermore, use of expensive aprotic polar organic solvent results in high cost.

### SUMMARY OF THE INVENTION

An object of the present invention is providing a method for preparing a hydroxybenzoic acid compound with high yield without using an expensive aprotic polar organic solvent.

The present invention provides a method for producing a hydroxybenzoic acid compound comprising, dehydrating a phenol compound and an alkaline metal compound to form an alkaline metal salt of phenol and reacting the alkaline metal salt and carbon dioxide, wherein the dehydrating step is conducted by reacting said alkaline metal compound with an excess amount of the phenol compound, which is in excess of the alkaline metal compound, at a temperature of 160°C or above.

According to the method of the present invention, by using an excess amount of the phenol compound over the alkaline metal compound, the dehydration between the phenol compound and the alkaline metal compound is promoted and consequently, the alkaline metal salt of the phenol compound can be obtained with high yield and the remaining phenol can be used again as a solvent.

In the present specification and claims, "excess" amount of the phenol compound means that the amount of the phenol compound is two or more molar parts per 1 molar part of the alkaline metal compound. In the present invention, the amount of the phenol compound is preferably 2-30 molar parts, more preferably 3-15 molar parts and even more preferably 4-10 molar parts per 1 molar part of the alkaline metal compound. In case where the amount of the phenol compound is less than 2 molar parts, the alkaline metal salt of the phenol compound will precipitate and interfere with homogeneous stirring. Using more than 30 molar parts of phenol is permissible, but it will not bring about better result than using less amount of phenol and therefore, it is not economical.

In the method of the present invention, dehydrating of the alkaline metal compound and the phenol compound is conducted at a temperature of 160°C or above and preferably at 180-300°C. If the temperature during dehydration is lower than 160°C, the alkaline metal salt hardly be formed and water produced by the dehydration of the phenol and the alkaline metal compound can not be sufficiently removed. On the contrary, if the temperature during the dehydration step is higher than 300°C which is above the boiling point of the phenol, the phenol might be distilled out of the reaction system and the alkaline metal salt of phenol might be thermally decomposed due to such a high temperature.

In the present invention, the phenol compound preferably used as a starting material is represented by formula (I): 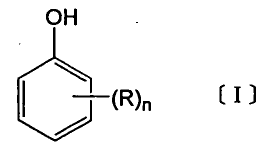 wherein, R is selected from the group consisting of hydrogen atom, linear or branched chain C1-20 alkyl, C1-20 alkenyl and C1-20 alkoxy groups; n is an integer from 1 to 4.

Among the above, alkyl substituted phenols (R= alkyl group), preferably dialkyl substituted phenols are suitably used for the method of the present invention because of their high reaction selectivity and high reaction yield. Examples of alkyl groups suitable for the substituents are methyl, ethyl, n-propyl, iso-propyl, n-butyl, sec-butyl, tert-butyl, n-octyl and tert-octyl.

Examples of alkyl substituted phenols preferably used in the present invention are o-cresol, p-cresol, m-cresol, 2,6-dimethylphenol, 3,5-dimethylphenol, 2,5-dimethylphenol, o-isopropylphenol, 2,6-di-tert-butylphenol, 2,4-di-tert-butylphenol, 2,5-di-tert-butylphenol, 4-n-octylphenol and 4-tert-octylphenol.

In case there are two or more substituents on the phenol, the substituents may be the same or different.

A preferable alkaline metal compound used in the present invention is sodium hydroxide or potassium hydroxide. In particular, sodium hydroxide is preferable because it allows rapid dehydration and is inexpensive.

In the method of the present invention, water produced during the dehydrating step is preferably removed from the reaction system.

In order to promote the dehydrating step, an azeotropic dehydration agent may be added to the reaction mixture. In general, one or more of hydrocarbon type solvents which are selected from the group consisting of aliphatic hydrocarbons such as octane, nonane, decane, undecane, dodecane, ligroin and kerosene; aromatic hydrocarbons such as benzene, toluene, xylene, mesitylene, ethylbenzene, cumene, diphenylether and naphthalene; and halogenated hydrocarbons such as chlorobenzene, o-dichlorobenzene, and p-dichlorobenzene are used as an azeotropic dehydration agent. The amount of the azeotropic dehydration agent used in the reaction may vary depending on the amount of water contained in the reaction system, and in general, the amount of the azeotropic dehydration agent used may be 2-10 parts by weight per 1 part by weight of water contained in the system.

In the method of the present invention, the liquid state substituted phenol acts as solvent and therefore, it is not necessary to add any other solvent in the step to obtain an alkaline metal salt of the phenol compound. However, the case where an additional solvent other than the substituted phenol is used is also included in the scope of the present invention. Solvents other than the phenol compound used in the dehydrating step may be any of those other than aprotic polar organic solvents. Examples of such solvents are light oil, kerosene, petrol, lubrication oil, white oil, alkylbenzene, alkylnaphthalene, diphenyl, diphenylalkane, alkyldiphenyl, triphenyl, hydrogenated triphenyl, diphenylether, alkylphenylether, alkyldiphenylether, high boiling point higher alcohols such as iso-octyl alcohol and a mixture thereof.

In the method of the present invention, dehydrating step is carried out under inert gases atmosphere such as nitrogen, helium and argon gas.

In the method of the present invention, the alkaline metal salt of the phenol compound obtained by the dehydrating step is subjected to the next step, i.e. reacting the same with carbon dioxide.

The reaction of the alkaline metal salt of the phenol compound and carbon dioxide is curried out in an autoclave under the carbon dioxide pressure of preferably 2.0-10 kgf/cm² (G), more preferably 4.0-8.0 kgf/cm² (G) at a reaction temperature preferably of 160-300°C, and more preferably of 170-290°C. The reaction time may vary depending on the carbon dioxide pressure and the reaction temperature, and in general, it may be 1-6 hrs, and preferably 1-4 hrs.

Into thus obtained reaction mixture comprising the alkaline metal salt of the hydroxybenzoic acid compound, water is added and then the mixture is separated into the solvent and water phases. Next, the water phase, i.e. an aqueous solution containing the alkaline metal salt of the hydroxybenzoic acid compound is added with an acid to precipitate the hydroxybenzoic acid compound. The precipitates may be collected by filtration and centrifugation to give crystalline hydroxybenzoic acid compound.

The solvent phase separated from the reaction mixture in this example is mainly consisting of the starting phenol compound and therefore, the solvent phase may be used again as starting phenol compound directly or, if necessary, after purified by, such as, filtration, distillation or carbon treatment.

Non limiting examples of the hydroxybenzoic acid compounds produced by the method of the present invention are 3,5-di-tert-butyl-4-hydroxybenzoic acid, 3,5-di-tert-butyl-2-hydroxybenzoic acid, 3-methyl-4-hydroxybenzoic acid, 2,6-dimethyl-4-hydroxybenzoic acid, 2-ethyl-4-hydroxybenzoic acid and 3,5-diethyl-4-hydroxybenzoic acid. Among the above, 3,5-di-tert-butyl-4-hydroxybenzoic acid produced from 2,6-di-tert-butylphenol and 3,5-di-tert-butyl-2-hydroxybenzoic acid 2,4-di-tert-butylphenol are preferable because they are obtained with high yields.

According to the method of the present invention, highly pure hydroxybenzoic acid compound can be obtained with low amount of side-products and with high yields. Moreover, the method of the present invention makes it possible to produce the hydroxybenzoic acid compound at low cost with simple steps without using an expensive aprotic polar solvent. Furthermore, the solvent phase separated from the reaction mixture in the method contains little amount of by-products and therefore, it can be used again as the starting phenol compound.

The hydroxybenzoic acid compound produced by the method of the present invention can be used as a raw material for producing ultraviolet absorber, antioxidant and the like which are contained in plastics such as polypropylene.

The present invention is further described in reference to the following examples. The examples are intended to illustrate the invention and are not to be construed to limit the scope of the invention.

### Example 1

432.6 g (2.1 moles)of 2,6-di-tert-butylphenol and 25g (0.3 mole) of 48% aqueous sodium hydroxide were fed in an autoclave having 1L stainless-steel vessel equipped with a magnetic stirrer, a thermometer, pressure gauge and water-separator. The reaction mixture was heated to 210°C under nitrogen gas flow and at this temperature, dehydration was conducted for 4 hrs. Next, nitrogen gas in the vessel was replaced with carbon dioxide gas and carboxylation was carried out at 210°C under the pressure of 6 kgf/cm² (G) with stirring for 2 hrs. After the reaction was completed, the reaction mixture was cooled to 60°C and 800 g of water was added thereto. The obtained mixture was heated to 65 °C and the mixture was separated into the water and solvent phases.

To thus obtained water phase, 73% aqueous sulfuric acid was added to precipitate crystal and the crystal was filtrated, washed with water and dried. As a result, 60g of powdery 3,5-di-tert-butyl-4-hydroxybenzoic acid was obtained. The yield to the fed amount of sodium hydroxide was 80%.

### Example 2

To the solvent phase removed from the water phase in example 1, 51.5 g (0.25 mole) of 2,6-di-tert-butylphenol and 25g (0.3 mole) of 48% aqueous sodium hydroxide were added. According to the similar method with example 1, 59.3g powdery 3,5-di-tert-butyl-4-hydroxybenzoic acid was obtained. The yield to the fed amount of sodium hydroxide was 79%.

It was confirmed that the solvent phase separated from the reaction mixture in example 1 did contain little amount of side-products and could be used again as starting material of the method of the present invention.

### Example 3

By the same method as Example 1 except that dehydrating step was conducted at 180°C, 37.5 g of powder of 3,5-di-tert-butyl-4-hydroxybenzoic acid was obtained. The yield to the fed amount of sodium hydroxide was 50%.

### Comparative Example 1

The same method as Example 1 was carried out except that dehydrating step was conducted at 150°C. In the comparative example 1, no precipitation was generated by adding 73% aqueous sulfuric acid and 3,5-di-tert-butyl-4-hydroxybenzoic acid could not be obtained.

### Example 4

The same method as Example 1 was carried out except that 2,4-di-tert-butylphenol was used instead of 2,6-di-tert-butylphenol, 60g of powdery 3,5-di-tert-butyl-2-hydroxybenzoic acid was obtained. The yield to the fed amount of sodium hydroxide was 80%.

### INDUSTRIAL APPLICABILITY

The present invention provides a method for producing highly pure hydroxybenzoic acid compound with high yield and little side-products. The present invention makes it possible to produce hydroxybenzoic acid compound by simple steps at low cost without using expensive aprotic polar organic solvent.

## Claims

1. A method for producing a hydroxybenzoic acid compound comprising, dehydrating a phenol compound and an alkaline metal compound to form the alkaline metal salt of the phenol compound and reacting the alkaline metal salt and carbon dioxide, wherein the dehydrating step is conducted by reacting the alkaline metal compound with an excess amount of the phenol compound, which is in excess of the alkaline metal compound, at a temperature of 160°C or above.

2. The method of claim 1, wherein said dehydrating step is conducted at a temperature of 180-300°C.

3. The method of claim 1, wherein 2-30 parts by mole of the phenol compound is reacted per 1 part by mole of the alkaline metal compound.

4. The method of claim 1, wherein the phenol compound is a compound represented by formula (I): 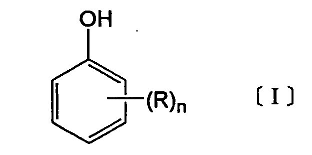 wherein, R is selected from the group consisting of hydrogen atom, linear or branched chain C1-20 alkyl, C1-20 alkenyl and C1-20 alkoxy groups; n is an integer from 1 to 4.

5. The method of claim 1, wherein the phenol compound is an alkyl substituted phenol.

6. The method of claim 1, wherein the phenol compound is a di-alkyl substituted phenol.

7. The method of claim 5 or 6, wherein, the alkyl group of the alkyl substituted phenol is selected from the group consisting of methyl, ethyl, n-propyl, iso-propyl, n-butyl, sec-butyl, tert-butyl and octyl.

8. The method of claim 1, wherein the phenol compound is selected from the group consisting of o-cresol, p-cresol, m-cresol, 2,6-dimethylphenol, 3,5-dimethylphenol, 2,5-dimethylphenol, o-isopropylphenol, 2,6-di-tert-butylphenol, 2,4-di-tert-butylphenol, 2,5-di-tert-butylphenol, 4-n-octylphenol and 4-tert-octylphenol.

9. The method of claim 1, wherein the phenol compound is 2,6-di-tert-butylphenol or 2,4-di-tert-butylphenol and said hydroxybenzoic acid is 3,5-di-tert-butyl-4-hydroxybenzoic acid or 3,5-di-tert-butyl-2-hydroxybenzoic acid.

10. The method of claim 1, wherein the alkaline metal compound is sodium hydroxide or potassium hydroxide.
